# EUROPEAN PATENT APPLICATION

(11) **EP 2 843 044 A1**
(43) Date of publication of application: **04.03.2015**
(21) Application number: 13306195.2
(22) Date of filing: 03.09.2013
(51) Int. Cl.: C12N 9/90, A23L 1/09

(54) **Improved variant of D-psicose 3-epimerase and uses thereof**

(71) Applicant: Roquette Frères, 62136 Lestrem (FR)
(72) Inventor: Lanos, Pierre, 59480 La Bassee (FR); Zhou, Liuming, Geneva, IL 60134 (US); Jia, Min, Dezhou City Shandong 251100 (CN); Zhang, Wenli, Tengzhou city Shandong 277500 (CN); Jiang, Bo, Jiangsu 214063 (CN); Mu, Wanmeng, Wuxi city Jiangsu (CN); Zhang, Tao, Wuxi city Jiangsu (CN)
(74) Representative: Gallois, Valérie

(57) **Abstract**

The present invention relates to an improved variant of a D-psicose 3-epimerase and its uses.

## Description

### FIELD OF THE INVENTION

The present invention relates to an improved isomerase, in particular D-psicose-3-epimerase, for preparing psicose from fructose and its uses.

### BACKGROUND OF THE INVENTION

D-psicose, also called D-allulose, is a rare sugar isomer of fructose. It can be found in nature but at very low concentrations like in edible mushrooms or the jackfruit, in wheat and the *Itea* plants.

At the opposite of fructose, the metabolism of psicose in humans is partly absorbed and metabolized in energy, and partly excreted unchanged in the urine and in the faeces.

The characteristics of D-psicose as a material for preventing lifestyle-related diseases have been disclosed, including its noncaloric nature, a positive effect on the reduction of the glycemic response, an antiobesity effect, and the like. In addition, the sweetness of D-psicose is about 70 % of that of sucrose (Oshima, et al. (2006) Psicose contents in various food products and its origin. Food Sci Technol Res 12:137-143), but 0.3% energy of sucrose and is suggested as an ideal sucrose substitute for food products. It can also be used as an inhibitor of hepatic lipogenic enzyme and intestinal α-glycosidase for reducing body fat accumulation. It further shows important physiological functions, such as reactive oxygen species scavenging activity and neuroprotective effect. In addition, it also improves the gelling behavior and produces good flavor during food process.

D-psicose exists in extremely small quantities in commercial carbohydrate or agricultural products and is difficult to chemically synthesize. Therefore, interconversion between D-fructose and D-psicose by epimerization using D-tagatose 3-epimerase (DTEase) family enzymes has been confused on as attractive way of D-psicose production.

So far, there have been 9 kinds of DTEase family enzyme sources reported. Twenty years ago, DTEase was firstly characterized by Izumori et al. from *Pseudomonas cichorii,* showing C-3 epimerization activity of ketohexoses with the optimum substrate of D-tagatose (Izumori et al. 1993, Biosci. Biotechnol. Biochem. 57, 1037-1039). Till 2006, the second enzyme with C-3 epimerization activity of ketohexoses was identified from *Agrobacterium tumefaciens,* and it was named D-psicose 3-epimerase (DPEase), due to its high substrate specificity for D-psicose (Kim et al. 2006, Applied and environmental microbiology 72, 981-985; US 2010/0190225, WO2011/040708). Recently, another six DTEase family enzymes were characterized from *Rhodobacter sphaeroides* SK011 (DTEase) (Zhang et al. 2009, Biotechnology letters 31, 857-862), *Clostridium cellulolyticum* H10 (DPEase) (Mu et al. 2011, Journal of agricultural and food chemistry 59, 7785-7792, CN102373230), *Ruminococcus* sp. 5_1_39BFAA (DPEase) (Zhu et al. 2012, Biotechnology letters 34, 1901-1906), *Clostridium bolteae* ATCC BAA-613 (Jia et al. 2013, Applied Microbiology and Biotechnology DOI 10.1007/s00253-013-4924-8), *Clostridium scindens* ATCC 35704 (Zhang et al. 2013, PLoS ONE 8, e62987), and *Clostridium* sp. BNL1100 (Mu et al. 2013, Biotechnology Letter DOI 10.1007/s10529-013-1230-6), respectively. In addition, Maruta et al. disclosed a DTEase producing source in *Rhizobium* (US 2011/0275138).

There is only one reference to report the enzyme modification of DTEase family enzymes by protein engineering technology. Using random and site-directed mutagenesis technology, Choi et al. (2011, Applied and environmental microbiology 77, 7316-7320) constructed the I33L S213C double-site variant of *A. tumefaciens* DPEase, and the variant enzyme showed increases in optimal temperature, half-life, melting temperature, and the catalysis efficiency, compared with the wild-type enzyme. Its optimal pH remains unchanged at 8.00.

However, the enzymes have optimum pH for activity at 8.0 - 9.5, and the pH stability is between 8.0 - 10.0, which is not appropriate for industrial application.

Therefore, the main concern for using psicose remains its scarcity and its production cost and the need for improved industrial D-psicose production still exists.

### SUMMARY OF THE INVENTION

To develop industrial D-psicose production and reduce the production cost, an optimized DTEase family enzyme should be weak-acid stable and thermostable, and has a higher catalysis efficiency and turnover for the substrate D-fructose.

The present invention relates to a variant of a parent D-psicose 3-epimerase, wherein the variant comprises a substitution of a glycine residue by a serine residue at a position corresponding to the position 211 in SEQ ID No 2 compared to the parent D-psicose 3-epimerase; and wherein the variant has a D-psicose 3-epimerase activity.

In a preferred embodiment, the variant has one or several following features:
a. a lower pH optimum compared to the parent D-psicose 3-epimerase, preferably in the range of 6 to 7; and/or
b. a higher catalysis efficiency to the substrate D-fructose compared to the parent-psicose 3-epimerase, preferably at least twice higher; and/or
c. a longer half-life at 60°C compared to the parent-psicose 3-epimerase.

Preferably, the variant has an amino acid sequence having 35 % of identity or higher with SEQ ID No 2, preferably 60 % of identity or higher, more preferably at least 70, 75, 80, 85, 90, 95 % of identity or higher.

In a preferred embodiment, the parent D-psicose 3-epimerase is selected from a D-tagatose 3-epimerase from *Pseudomonas cichorii,* a D-psicose 3-epimerase from *Agrobacterium tumefaciens,* a D-psicose 3-epimerase from *Clostridium sp,* a D-psicose 3-epimerase from *Clostridium scindens,* a D-psicose 3-epimerase from *Clostridium bolteae,* a D-psicose 3-epimerase from *Ruminococcus sp,* and a D-psicose 3-epimerase from *Clostridium cellulolyticum.* More preferably, the parent D-psicose 3-epimerase is the D-psicose 3-epimerase from *Clostridium cellulolyticum.* In a most preferred embodiment, the variant comprises or consists of the amino acid sequence of SEQ ID No 4 or an amino acid sequence having 90 or 95 % of identity with SEQ ID No 4 or higher and having a residue serine at position 211.

Another object of the present invention is an isolated nucleic acid encoding a variant according to the present invention. The present invention further relates to an expression cassette or recombinant expression vector comprising a nucleic acid encoding a variant according to the present invention. It also relates to a recombinant host cell comprising a nucleic acid according to the present invention, an expression cassette according to the present invention or a recombinant expression vector according to the present invention. In a particular embodiment, the host cell is a GRAS strain (Generally Recognized As Safe), preferably *Bacillus subtilis.*

The present invention relates to a method for producing a D-psicose 3-epimerase variant comprising culturing the recombinant host cell according to the present invention, and optionally recovering or purifying the produced D-psicose 3-epimerase variant from the resulting culture. In other word, it relates to the use of a recombinant host cell according to the present invention for producing a D-psicose 3-epimerase variant according to the present invention.

The present invention also relates to a method for producing D-psicose comprising contacting a variant according to the present invention with D-fructose in conditions suitable for the D-psicose 3-epimerase activity and optionally recovering the produced D-psicose. Optionally, the D-fructose is previously or simultaneously produced by a glucose isomerase from D-glucose. Then, the present invention relates to the use of a D-psicose 3-epimerase variant according to the present invention or a recombinant host cell according to the present invention for producing D-psicose.

An object of the present invention is an enzymatic composition comprising a D-psicose 3-epimerase variant according to the present invention and an additional enzyme, in particular a glucose isomerase.

Finally, the present invention relates to the use of a GRAS host cell according to the present invention for preparing a food product and to a food product comprising such a GRAS host cell.

### DETAILED DESCRIPTION OF THE INVENTION

The present invention relates to an improved variant of a D-psicose 3-epimerase.

### Definitions

In the present document, the term "DPEase" and "DTEase" could be used in place of "D-psicose 3-epimerase" and "D-tagatose 3-epimerase", respectively. And "DPEase" and "DTEase" mean the ketose 3-epimerases with the optimum substrates as D-psicose and D-tagatose, respectively. In addition, the term "DPEase variant" may also referred to a variant of a D-tagatose 3-epimerase as taught in the present invention.

Identity Percentage: The "percentage identity" between two amino acid sequences (A) and (B) is determined by comparing the two sequences aligned in an optimal manner, through a window of comparison. Said alignment of sequences can be carried out by well-known methods, for example, using the algorithm for global alignment of Needleman-Wunsch. Protein analysis software matches similar sequences using measures of similarity assigned to various substitutions, deletions and other modifications, including conservative amino acid substitutions. Once the total alignment is obtained, the percentage of identity can be obtained by dividing the full number of identical amino acid residues aligned by the full number of residues contained in the longest sequence between the sequence (A) and (B). Sequence identity is typically determined using sequence analysis software. For comparing two amino acid sequences, one can use, for example, the tool "Emboss needle" for pairwise sequence alignment of proteins providing by EMBL-EBI and available on www.ebi.ac.uk/Tools/services/web/toolform.ebi?tool=emboss_needle&context=protein, using default settings : (I) Matrix : BLOSUM62, (ii) Gap open : 10, (iii) gap extend : 0.5, (iv) output format : pair, (v) end gap penalty : false, (vi) end gap open : 10, (vii) end gap extend : 0.5.

By "about" is intended the value more or less 10 % of the value. Preferably, it is intended the value more or less 5 % of the value. For instance, "about 100" means between 90 and 110, preferably between 95 and 105.

By "D-psicose 3-epimerase activity" is referred the capacity of the enzyme to modify D-fructose into D-psicose. This activity can be assayed by measuring the amount of D-psicose formed from D-fructose. In particular, it can be measured as detailed in the Example section or as disclosed in Mu et al. (2011, Journal of agricultural and food chemistry 59, 7785-7792; in "Enzyme Assay" section page 7787).

### Variant of D-psicose 3-epimerase

The present invention relates to a variant of a parent D-psicose 3-epimerase, wherein the variant comprises a substitution of a glycine residue by a serine residue at a position corresponding to the position 211 in SEQ ID No 2 compared to the parent D-psicose 3-epimerase; and wherein the variant has a D-psicose 3-epimerase activity.

The inventors surprisingly identified a G211S variant of DPEase from *C. cellulolyticum* as an improved variant. Indeed, this variant presents the following advantages (see Tables 1 and 2):
- a lower pH optimum, namely 6.5 instead of 8.0 for the wild-type DPEase;
- a higher half-life at 60°C, namely 7.2 h instead of 6.8; and
- a higher k_{cat}/Kₘ for D-fructose, namely 150.6 instead of 62.7.

According to the knowledge of the inventors, it is the first time that a DPEase is reported with a pH optimum lower than 7.0. In addition, the lowering of the pH optimum goes along with an improved stability and a strong increase of catalytic efficiency.

Accordingly, the DPEase variant has one or several following features:
a. a lower pH optimum compared to the parent D-psicose 3-epimerase, preferably in the range of 6 to 7; and/or
b. a higher catalysis efficiency to the substrate D-fructose compared to the parent-psicose 3-epimerase, preferably at least 50, 75, 100, 120 % higher, more preferably at least twice higher; and/or
c. a longer half-life at 60°C, preferably of at least 5, 10, 15 or 20 minutes longer.

In a first embodiment, the DPEase variant fulfils the requirement of items a) and b), items a) and c), items b) and c), or items a), b) and c). Preferably, the DPEase variant has a lower pH optimum compared to the parent D-psicose 3-epimerase, preferably in the range of 6 to 7. Therefore, the DPEase variant fulfils the requirement of items a) and b), items a) and c), or items a), b) and c).

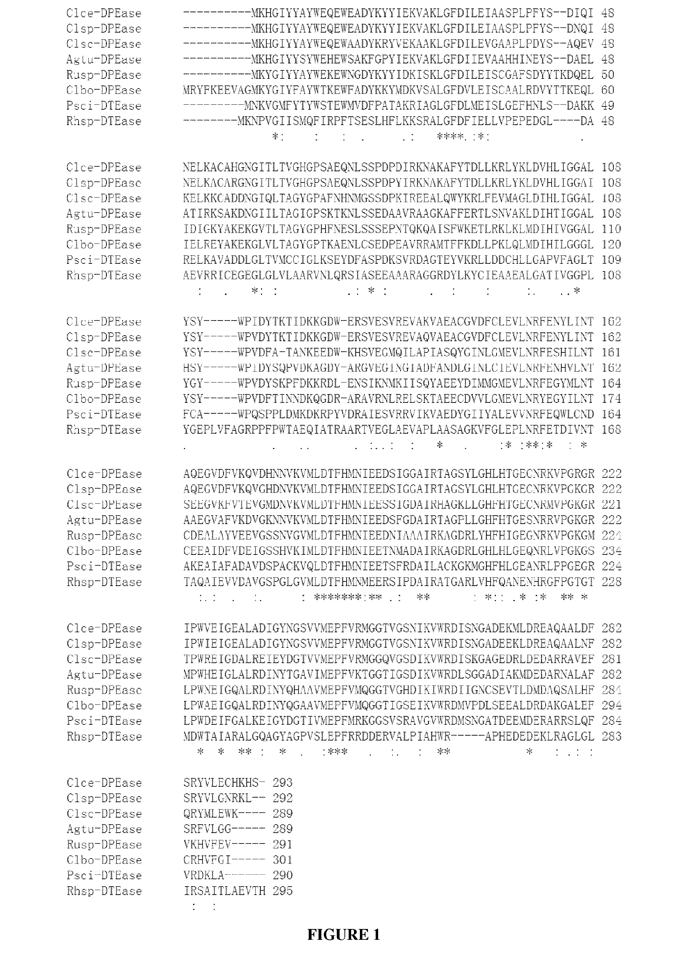

The inventors further noted that, despite a quite low amino acid (aa) sequence identity between D-tagatose 3-epimerase from *Pseudomonas cichorii,* D-psicose 3-epimerase from *Agrobacterium tumefaciens,* and D-psicose 3-epimerase from *Clostridium cellulolyticum* (i.e., DTEase of *P. cichorii* has 41 % aa identity with DPEase of *C. cellulolyticum;* DPEase of *A. tumefaciens* has 60 % aa identity with DPEase of *C. cellulolyticum*)*,* the residue G211 of the DPEase of *C. cellulolyticum* is conserved. Furthermore, as shown in Figure 1, G211 is conserved seven of the eight enzymes (see Figure 1).

Then, the present invention relates to a DPEase variant having an amino acid sequence having 35 % of identity or higher with SEQ ID No 2, preferably 60 % of identity or higher, more preferably at least 70, 75, 80, 85, 90, 95 % of identity or higher. It is obviously understood that all the DPEase variants of the present invention present the substitution of Gly by Ser at the position corresponding to the residue 211 in SEQ ID No 2.

More particularly, the parent D-psicose 3-epimerase is selected from a D-tagatose 3-epimerase from *Pseudomonas cichorii,* a D-psicose 3-epimerase from *Agrobacterium tumefaciens,* a D-psicose 3-epimerase from *Clostridium sp,* a D-psicose 3-epimerase from *Clostridium scindens,* a D-psicose 3-epimerase from *Clostridium bolteae,* a D-psicose 3-epimerase from *Ruminococcus sp,* and a D-psicose 3-epimerase from *Clostridium cellulolyticum.* In a preferred embodiment, the parent D-psicose 3-epimerase is a D-psicose 3-epimerase from *Clostridium cellulolyticum,* more particularly *Clostridium cellulolyticum* strain H10 (ATCC 35319).

Therefore, the present invention relates to a DPEase variant having or comprising the amino acid sequence of SE ID No 2 with a G211S substitution (i.e., the amino acid sequence of SEQ ID No 4) or an amino acid sequence having 90 or 95 % of identity with SEQ ID No 4 or higher and having a residue Ser at position 211.

Alternatively, it also relates to a DPEase variant having or comprising the amino acid sequence of SE ID No 5 with a G211S substitution or an amino acid sequence having 90 or 95 % of identity with SEQ ID No 5 or higher and having a residue Ser at position 211.

In addition, it also relates to a DPEase variant having or comprising the amino acid sequence of SE ID No 6 with a G210S substitution or an amino acid sequence having 90 or 95 % of identity with SEQ ID No 6 or higher and having a residue Ser at position 210.

Alternatively, it also relates to a DPEase variant having or comprising the amino acid sequence of SE ID No 7 with a G211S substitution or an amino acid sequence having 90 or 95 % of identity with SEQ ID No 7 or higher and having a residue Ser at position 211.

In addition, it also relates to a DPEase variant having or comprising the amino acid sequence of SE ID No 8 with a G213S substitution or an amino acid sequence having 90 or 95 % of identity with SEQ ID No 8 or higher and having a residue Ser at position 213.

Alternatively, it also relates to a DPEase variant having or comprising the amino acid sequence of SE ID No 9 with a G223S substitution or an amino acid sequence having 90 or 95 % of identity with SEQ ID No 7 or higher and having a residue Ser at position 223.

Finally, it also relates to a DTEase variant having or comprising the amino acid sequence of SE ID No 10 with a G213S substitution or an amino acid sequence having 90 or 95 % of identity with SEQ ID No 10 or higher and having a residue Ser at position 213.

Optionally, the variant has alterations at not more than 11, 10, 9, 8, 7, 6, 5, 4, 3, 2 or 1 amino acids, e.g., may have substitution, insertion, and/or deletion of 1, 2, 3, 4, 5, 6, 7, 8, 9 or 10 amino acids.

The present invention also relates to a DPEase variant according to the present invention further comprising a tag. For instance, it can comprise tag suitable for facilitate the DPEase variant purification or immobilization, such as a His tag (His₆), a FLAG tag, a HA tag (epitope derived from the Influenza protein haemagglutinin), a MYC tag (epitope derived from the human proto-oncoprotein MYC) or a GST tag (small glutathione-S-transferase).

Finally, the present invention relates to a DPEase variant according to the present invention immobilized on a solid support or a carrier. The DPEase can be immobilized on any suitable support or carrier, such as alginate, amberlite resin, Sephadex resin or Duolite resin, e.g., beads. Immobilization means are well-know to the person skilled in the art. For instance, see Choi et al, *supra*; Lim et al. (2009, Porcess Biochemistry 44, 822-828) and WO2011/040708, the disclosure thereof being incorporated herein by reference.

### Nucleic acid, vector and host cells

The present invention relates to a nucleic acid encoding a DPEase variant according to the present invention or a nucleic acid comprising a sequence encoding a DPEase variant according to the present invention. The present invention also relates to an expression cassette of a nucleic acid according to the invention. It further relates to a vector comprising a nucleic acid or an expression cassette according to the invention. Preferably, the vector is an expression vector. The vector is preferably a plasmid vector. In addition, the present invention relates to a host cell comprising a nucleic acid according to the invention, an expression cassette of a nucleic acid according to the invention or a vector comprising a nucleic acid or an expression cassette according to the invention. The nucleic acid encoding a DPEase variant according to the present invention can be present in the host cell as an episomic sequence or can be incorporated into its chromosome. The nucleic acid encoding a DPEase variant according to the present invention can be present in the host cell in one copy or in several copies.

The nucleic acid can be DNA (cDNA or gDNA), RNA, or a mixture of the two. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, mutagenesis etc...

The expression cassette comprises all elements required for expression of the DPEase variant according to the present invention, in particular the elements required for transcription and translation in the host cell, in particular in the considered host cell.

The host cell can be prokaryotic or eukaryotic, preferably prokaryotic or lower eukaryotic, more preferably prokaryotic. In particular, the expression cassette comprises a promoter and a terminator, optionally an enhancer. The promoter can be prokaryotic or eukaryotic, depending on the selected host cell. Examples of preferred prokaryotic promoters include: Lacl, LacZ, pLacT, ptac, pARA, pBAD, the RNA polymerase promoters of bacteriophage T3 or T7, the polyhedrin promoter, the PR or PL promoter of lambda phage. In general, to select a suitable promoter, one skilled in the art may advantageously consult Sambrook et al. work (1989) or techniques described by Fuller et al. (1996; Immunology in Current Protocols in Molecular Biology).

The present invention relates to a vector containing a nucleic acid or an expression cassette encoding the DPEase variant according to the present invention. The vector is preferably an expression vector, that is to say, it comprises the elements required for the expression of the variant in the host cell. The vector is a self-replicable vector. The host cell can be a prokaryote, for example *E. coli,* or a eukaryote. The eukaryote can be a lower eukaryote such as a yeast (for example, *S. cerevisiae*) or fungus (for example from the genus *Aspergillus* or *Actinomyces*) or a higher eukaryote such as an insect, mammalian or plant cell. The cell can be a mammalian cell, for example COS, CHO (US 4,889,803; US 5,047,335). In a particular embodiment, the cell is non-human and non-embryonic.

The vector can be a plasmid, phage, phagemid, cosmid, virus, YAC, BAC, pTi plasmid from *Agrobacterium,* etc... The vector can preferably comprise one or more elements selected from the group consisting of a replication origin, a multiple cloning site and a selection gene. In a preferred embodiment, the vector is a plasmid. The vector is a self-replicable vector. Examples of prokaryotic vectors include, but are not limited to, the following: pER322, pQE70, pMA5, pUC18, pQE60, pUB110, pQE-9 (Qiagen), pbs, pTZ4, pC194, pD10, pHV14, Yep7, phagescript, psiX174, pbluescript SK, pbsks, pNH8A, pNH16A, pNH18A, pNH46A (Stratagene); ptrc99a, pKK223-3, pKK233-3, pDR540, pBR322, and pRIT5 (Pharmacia), pET (Novagen). Examples of eukaryotic vectors include, but are not limited to, the following: pWLNEO, pSV2CAT, pPICZ, pcDNA3.1 (+) Hyg (Invitrogen), pOG44, pXT1, pSG (Stratagene); pSVK3, pBPV, pCI-neo (Stratagene), pMSG, pSVL (Pharmacia); and pQE-30 (QLAexpress). Preferably the expression vector is a plasmid vector.

More particularly, to express in *E. coli,* pBR322, pUC18, pBluescript II SK (+), λgt.λC and λgt.λB can be preferably used. While, to express in *Bacillus subtilis,* pUB110, pTZ4, pC194, p11, Φ1 and Φ105 can be preferably used. Plasmids, pHV14, TRp7, YEp7 and pBS7 are useful in the case of replicating the recombinant nucleic acid in two or more kinds of hosts. In order to insert encoding nucleic acid sequence into these vectors, conventional methods in the art can be used.

In a particular embodiment, the vector is an integration vector suitable to incorporate the sequence encoding the DPEase variant according to the present invention into the chromosome of the host cell. A non-exhaustive example of commercially available integration vectors is pMUTIN4 for *B. substilis* from Bacillus Genetic Stock Center.

The host cell can be preferably selected among the group consisting of *E. coli,* and GRAS strains. Preferably, the GRAS strain is selected from the group consisting of innocuous bacteria, especially innocuous *Corynebacterium sp.* such as *C*. *glutamicum,* and innocuous *Bacillus sp.* such as *B. subtilis.* In a very specific embodiment, the host cell is of *E. coli* or *B. subtilis,* preferably *B. Subtilis.*

The present invention relates to the use of a nucleic acid, an expression cassette, an expression vector or a host cell as disclosed above for producing a DPEase variant according to the present invention.

It also relates to a method for producing a DPEase variant according to the present invention comprising culturing the recombinant host cell according to the present invention, and optionally recovering and/or purifying the produced D-psicose 3-epimerase variant from the resulting culture. In a preferred embodiment, the host cell is selected from *E. coli,* and GRAS strains, especially *B. subtilis.*

Optionally, the host cell further produces a glucose isomerase.

In a particular embodiment, the present invention relates to an immobilized host cell according to the present invention producing and secreting a DPEase variant of the present invention.

### Production of D-psicose

The present invention relates to the use of a DPEase variant according the present invention for producing D-psicose and to the method for producing D-psicose by using a DPEase variant according the present invention.

In a first embodiment, the DPEase variant is contacted with D-fructose in conditions suitable for the D-psicose 3-epimerase activity. D-fructose can be provided as high fructose syrup, and in particular high fructose corn syrup. Such high fructose corn syrups are commercially available from Roquette Freres under HI-SWEET® references.

In another particular embodiment, D-glucose is contacted with an enzyme mixture comprising a DPEase variant according the present invention and a glucose isomerase. The glucose isomerase is also called xylose isomerase and corresponds to EC. 5.3.1.5. Preferably, glucose is provided as a glucose syrup, in particular a corn syrup.

In another alternative, the starting material may be starch in place of glucose or fructose, and the enzyme mixture further comprises alpha-amylase and/or glucoamylase.

The present invention relates to an enzyme mix comprising a DPEase variant according the present invention and an additional enzyme. Preferably, the enzyme mix comprises a DPEase variant according the present invention and a glucose isomerase. Optionally, the enzyme mix may further comprise alpha-amylase and/or glucoamylase.

Suitable conditions for producing D-psicose can be defined by the person skilled in the art. Preferably, they include the following features:
- Temperature: between 50 and 60°C, preferably about 55°C; and/or
- pH: between 5.5 and 7.5, preferably between 6 and 7, more preferably about 6.5; and/or
- in presence of a divalent metal ion, preferably selected from the group consisting of Co²⁺, Mn²⁺, Fe²⁺, Ni²⁺ and Mg²⁺, more preferably from Co²⁺, Mn²⁺, Fe²⁺, and Ni²⁺, still preferably from Co²⁺ and Mn²⁺, and most preferably Co²⁺; and/or
- in presence of borate when immobilized TDEase is used, e.g., 40-80 mM of borate, preferably about 60 mM .

In a particular embodiment, the enzymes to be used in the method are immobilized. More particularly, the DPEase variant of the present invention is immobilized. Optionally, both glucose isomerase and DPEase variant of the present invention can be immobilized or solely the DPEase variant. In another alternative, instead of immobilizing the enzyme, the microorganisms producing the enzymes are immobilized. The enzyme(s) or microorganisms can be for instance immobilized on any suitable support, such as alginate, amberlite resin, Sephadex resin or Duolite resin, e.g., beads.

The immobilized enzyme(s) or microorganisms can be packed into a suitable column and the glucose or fructose liquid or syrup is continuously introduced into the column.

Methods for immobilized DPEases on a support and to produce D-psicose are well known to the person skilled in the art, for instance in WO2011/040708.

The resulting product can be a mixture of D-fructose and D-psicose, and even a mixture of D-glucose, D-fructose and D-psicose.

An aspect of the present invention relates to the use of a GRAS host cell according to the present invention for preparing a food product and to a food product comprising such a GRAS host cell. The food products are for humans or for animal feed. For instance, foods can be foods for health, foods for patients, food materials, food materials for health, food materials for patients, food additives, food additives for health, food additives for patients, beverages, beverages for health, beverages for patients, potable water, potable water for health, potable water for patients, drugs, drug raw materials, feeds, and feeds for diseased domestic animals and/or diseased animals. When used as a food material or a food additive, it can be used for alleviating abnormal carbohydrate metabolism and/or abnormal lipid metabolism. It may be in the form of a solid preparation such as a tablet, a capsule, or a powder or granules to be dissolved in beverages, etc.; a semisolid preparation such as jelly; a liquid such as potable water; a high-concentration solution to be diluted before use; or the like.

### BRIEF DESCRIPTION OF THE DRAWINGS

**Figure 1****.** Multiple sequence alignment of DTEase family enzymes and their homologs. Amino acid sequence were from *C. cellulolyticum* DPEase (Clce-DPEase; GeneBank accession No: ACL75304), *Clostridium* sp. (Clsp-DPEase; YP_005149214.1), *C*. *scindens* DPEase (Clsc-DPEase; EDS06411.1), *A. tumefaciens* DPEase (Agtu-DPEase; AAL45544), *C*. *bolteae* DPEase (Clbo-DPEase; EDP19602), *Ruminococcus* sp. DPEase (Rusp-DPEase; ZP_04858451), *P. cichorii* DTEase (Psci-DTEase; BAA24429), and *R. sphaeroides* DTEase (Rhsp-DTEase; AC059490). The alignment was performed using ClustalW2 program (www.ebi.ac.uk/Tools/clustalw2/index.html). Amino acid residues that are identical in all the displayed sequences are marked by asterisks (*), strongly conserved or weakly conserved residues are indicated by colons (:) or dots (.), respectively.
**Figure 2****.** Comparison of pH profiles between wild-type and G211S mutant of *C. cellulolyticum* DPEase.
**Figure 3****.** SDS-PAGE analysis of purified G211S DTEase variant and protein markers stained with Coomassie blue. Lane 1, *Bacillus subtilis* producing DPEase; Lane 2, protein marker.

### EXAMPLES

The inventors prepared, by site-directed mutagenesis, DPEase variants of *C*. *cellulolyticum* by replacing the codon GGC encoding Gly in position 211 (SEQ ID No 1) by the codons AGC, GCC, GAC, CGC, TGG and CTC, encoding respectively the substitutions G211 S, G211A, G211D, G211T, G211W and G211L.

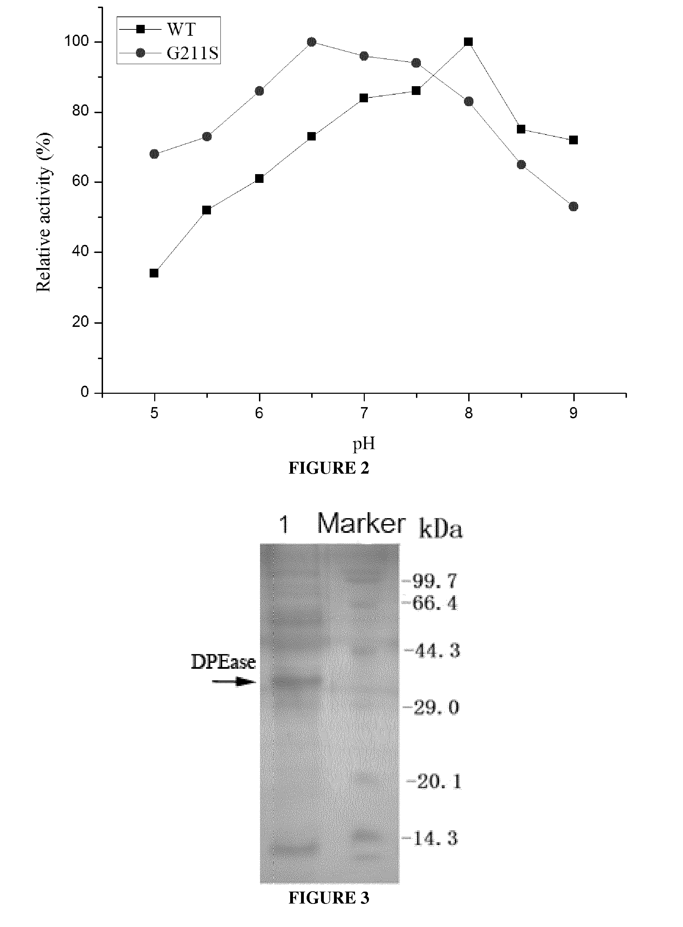

The DPEase variants have been expressed in *Bacillus subtilis,* expressed and purified (Figure 3).

Enzyme properties and kinetic parameters of the wild-type and variants of DPEase from C. *cellulolyticum* for substrate D-psicose have been determined and the results are given in Table 1.

The G211S variant showed improved characteristics in comparison with the wildtype DPEase (Table 1), but also with the other known enzymes (Table 2). In particular, it is a weak-acid stable enzyme with more than 80 % activity in the pH range from 6 to 8 (Figure 2), has a catalysis efficiency of approximately 150.6, and a half-life of at least 10 hours at 55 °C and/or a half life of at least 6.5 hours at 60 °C. In addition, the host is a food grade microorganism. These attributes are important in the bioproduction of a food-grade D-psicose with a more efficient production cycle and lower production costs.

### Materials and Methods

### Chemicals and reagents

Taq DNA polymerase, deoxynucleoside triphosphate (dNTP), chemicals for PCR, T4 DNA ligase and plasmid miniprep kit were obtained from Takara (Dalian, China). The resin for protein purification, the Chelating Sepharose Fast Flow, was obtained from GE (Uppsala, Sweden). Electrophoresis reagents were purchased from Bio-Rad. Isopropyl β-D-1-thiogalactopyranoside (IPTG) and all chemicals used for enzyme assays and characterization were at least of analytical grade obtained from Sigma (St Louis, MO, USA) and Sinopharm Chemical Reagent (Shanghai, China). Oligonucleotides were synthesized by Sangon Biological Engineering Technology and Services (Shanghai, China).

### Plamids, Bacterial strains, and Culture conditions

The plasmid pET-22b(+) was obtained from Novagen (Darmstadt, Germany). The *E*. *coli* DH5α and *E. coli* BL21(DE3) were obtained from Tiangen Biotechnology (Beijing, China). *Bacillus subtilis* WB600 and the plasmid pMA5 were obtained from Invitrogen (Carlsbad, CA, USA). The bacterial strains were grown in Luria-Bertani medium in a rotary shaker (200 rpm) at 37 °C.

### Preparation of DPEase variants of C. cellulolyticum in E.coli

(1) Primers design for protein modification was as following:
Forward mutagenic primers:
   G211S Forward primer1 :CATTTACACACTAGCGAATGTAATCGT (SEQ ID No 12)
   G211A Forward primer2 :CATTTACACACTGCCGAATGTAATCGT (SEQ ID No 13)
   G211D Forward primer3 :CATTTACACACTGACGAATGTAATCGT (SEQ ID No 14)
   G211R Forward primer4 :CATTTACACACTCGCGAATGTAATCGT (SEQ ID No 15)
   G211 W Forward primer5 :CATTTACACACTTGGGAATGTAATCGT (SEQ ID No 16)
   G211L Forward primer6 :CATTTACACACTCTCGAATGTAATCGT (SEQ ID No 17)
   Reverse primer: 5'-AGTGTGTAAATGTCCCAAGTAAGAGCCCGC-3' (SEQ ID No 18)
(2) Amplify the plasmid using the above primers by PCR technique.
Template: pET-Cc-dpe
DNA polymerase: Pfu
PCR program: PCR amplification was perfomed by Pfu DNA polymerase for 20 cycles consisting of 94 °C for 30s, 60 °C for 30 s, and 72 °C for 5 min, followed by extension step of 10 min at 72 °C.
(3) After PCR, add lul *Dpn*I restriction enzyme (10U/µl) into 200 µl PCR product, and incubate at 37 °C for 4 h, to digest and eliminate the template DNA.
(4) The DNA was purified by Gel Extraction Kit.
(5) The 5'-phosphorylation and ligation reactions of mutation fragments were performed together at 16 °C for 12h, and the reaction system was as follows:

| | |
|---|---|
| Mutation DNA | 7.5 µl |
| 10×T4 ligase buffer | 1 µl |
| PNK | 0.5 µl |
| T4 ligase | 1 µl |

(6) The DNA was transformed into *E. coli* DH5α. The transformants were selected at 37°C on the LB agar plates containing 100 µg/mL ampicillin.
(7) The plasmid was extracted and identified by nucleotide sequencing.
(8) The reconstructed plasmid was transformed in to *E. coli* BL21

The transformants were selected at 37°C on the LB agar plates containing 100 µg/mL ampicillin.

### Preparation of DPEase variants of C. cellulolyticum in B. subtilis

(1) PCR
   As to subclone the different variant genes to *B. subtilis* expression plasmid, forward (5'-CGCCATATGAAACATGGTATATACTACGC-3' - SEQ ID No 19) and reverse primer (5'-CGCGGATCCTTGTTAGCCGGATCTC-3' - SEQ ID No 20) were designed to introduce the *Nde*I and *Bam*HI restriction sites. Using the reconstructed pET-22b(+) plasmids harboring different DPEase variant genes, PCR amplification were seperately performed by Taq Plus DNA polymerase for 35 cycles consisting of 94 °C for 1 min, 60 °C for 1 min, and 72 °C for 1 min, followed by a final extension step of 10 min at 72 °C.
(2) Purify the PCR products seperately using the Gel Extraction Kit.
(3) The purified PCR products and *B. subtilis* expression plasmid, pMA5 were digested by restriction enzyme *Nde*I and *Bam*HI
(4) DNA fragment and pMA5 were ligated by T4 DNA Ligase, and then the mixture was transformed in to *E. coli* DH5α.
(5) The transformants were selected at 37 °C on the LB agar plates containing 100 µg/mL Ampicillin.
(6) The reconstructed plasmids were extracted and identified by restriction enzyme digestion and nucleotide sequencing.
(7) The reconstructed pMA5 plasmids harboring the wild-type or variant DPEase gene were seperately transformed in to *B. subtilis* WB600 by electroporation. The transformants were selected at 37°C on the LB agar plates containing 100 µg/mL Kanamycin.

### Purification of DPEase variants of C. cellulolyticum

To purify the recombinant DPEase variants, the centrifuged cell pellets were resuspended in lysis buffer (50 mM Tris-HCl, 100 mM NaCl, pH 7.5) and disrupted by sonication at 4 °C for 6 min (pulsations of 3 s, amplify 90) using a Vibra-Cell 72405 sonicator, and cell debris was removed by centrifugation (20000g, 20 min, 4 °C). The cell-free extract was applied onto a Chelating Sepharose Fast Flow resin column (1.0 cm x 10.0 cm), previously chelating Ni²⁺, and equilibrated with a binding buffer (50 mM Tris-HCl, 500 mM NaCl, pH 7.5). Unbound proteins were eluted from the column with a washing buffer (50 mM Tris-HCl, 500 mM NaCl, 50 mM imidazole, pH 7.5). Then, the DPEase variants were eluted from the column with an elution buffer (50 mM Tris-HCl, 500 mM NaCl, 500 mM imidazole, pH 7.5). The active fractions were pooled and dialyzed overnight against 50mMTris-HCl buffer (pH 7.5) containing 10 mM ethylenediaminetetraacetic acid (EDTA) for 48 h at 4 °C. Subsequently, the enzyme was dialyzed against 50 mM EDTA-free Tris-HCl buffer (pH 7.5).

### DPEase Assay

The activity was measured by the determination of the amount of produced D-psicose from D-fructose. The reaction mixture of 1 mL contained D-fructose (50 g/L), Tris-HCl buffer (50 mM, pH 8.0), 0.1mM Co²⁺, and 0.5 µM enzyme. The reaction mixture was incubated at 55 °C for 2 min, and the reaction was stopped after 10 min by boiling. The generated D-psicose was determined by the HPLC method. One unit of enzyme activity was defined as the amount of enzyme catalyzing the formation of 1 µmol of D-psicose/min at pH 8.0 and 55 °C.

### Effect of Temperature and pH

The optimum temperature of enzyme activity was measured by assaying the enzyme samples over the range of 35-70 °C for 2 min. Two buffer systems, sodium phosphate (50 mM, pH 6.0-7.0) and Tris-HCl (50 mM, pH 7.5-9.0), were used for measuring the optimum pH of enzyme activity. The thermal stability of the enzyme was studied by incubating the enzyme in Tris-HCl buffer (50 mM, pH 8.0) at various temperatures. At given time intervals, samples were withdrawn and the residual activity was measured under standard assay conditions. To determine the pH stability, the enzyme was incubated at pH 6.0-9.0 at 4 °C for up to 2 h, and the remaining enzyme activity was measured at time intervals under standard assay conditions.

### Determination of kinetic parameters

Kinetic parameters of DPEase variants were determined in 50 mM Tris-HCl buffer (pH 8.0) containing 0.1mM Co²⁺ and 5-200mM substrate for reaction at 55 °C. The enzyme reactions were stopped after 10 min by boiling, and the amount of D-psicose was determined by the HPLC assay. Kinetic parameters, such as the Michaelis-Menten constant (Kₘ) and turnover number (k_{cat}) values for substrates, were obtained using the Lineweaver-Burk equation and quantification of enzyme concentration.

### Analytical Methods

The concentrations of D-fructose and D-psicose were analyzed by HPLC equipped with a refractive index detector and a Ca²⁺-carbohydrate column (Waters Sugar-Pak 1, Waters Corp., Milford, MA), which was eluted with water at 85 °C and 0.4 mL/min. Protein concentration was determined according to the method of Bradford using bovine serum albumin as a standard. SDS-PAGE was carried out according to the method of Laemmli. Gels (12% w/v polyacrylamide) were stained with Coomassie Brilliant Blue and destained with an aqueous mixture of 10% (v/v) methanol/10% (v/v) acetic acid.

**Table 1: Enzyme properties and kinetic parameters of the wild-type and mutant enzymes of DPEase from C. cellulolyticum for substrate D-psicose**

| Enzyme | **Optimum pH** | Optimum temp. (°C) | Equilibrium ratio between D-psicose and D-fructose at 55 °C | **Half-life at 60 °C (h)** | ***k***_{cat}/***K***ₘ **for D-fructose (mM⁻¹ min ⁻¹)** |
|---|---|---|---|---|---|
| Wild type | **8.0** | 55 | 32:68 | **6.8** | **62.7** |
| G211S | **6.5** | 55 | 33:67 | **7.2** | **150.6** |
| G211A | **8.5** | 50 | 30:70 | **2.5** | **9.8** |
| G211D | **8.0** | 60 | 32:68 | **5.3** | **86.7** |
| G211R | **6.5** | 45 | 28:72 | **3.7** | **26.5** |
| G211W | **7.0** | 60 | 32 :68 | **5.9** | **68.1** |
| G211L | **8.0** | 55 | 30 :70 | **6.7** | **73.2** |

| | | | | | |
|---|---|---|---|---|---|
| *^{a}* NR, not reported. | | | | | |

**Table 2: Enzyme properties and kinetic parameters of DTEase family enzymes for D-psicose production**

| DTEase family enzymes | **Optimum pH** | Optimum temp. (°C) | Equilibrium ratio between D-psicose and D-fructose | **Half-life (thermostability)** | ***k***_{cat}/***K***ₘ **for D-fructose (mM⁻¹ min ⁻¹)** | Reference |
|---|---|---|---|---|---|---|
| *C. cellulolyticum* DPEase mutant of G211 S | **6.5** | 55 | 33:67 (55 °C) | **10.1 h (55°C)** | **150.6** | Invention |
| | | | | **7.2 h (60 °C)** | | |
| *C. cellulolyticum* DPEase | **8.0** | 55 | 32:68 (55 °C) | **9.5 h (55 °C)** | **62.7** | Mu et al. 2011 |
| | | | | **6.8 h (60 °C)** | | |
| *Clostridium* sp. DPEase | **8.0** | 65 | 28:72 (65 °C) | **0.25 h^{b} (60 °C)** | **58.7** | Mu et al. 2013 |
| *C. bolteae* | **7.0** | 55 | 32:68 (60 °C) | **2.6 h^{b} (55 °C)** | **59.4^{c}** | Jia et al. 2013 |
| *C*. *scindens Ruminococcus* | **7.5** | 60 | 28:72 (50 °C) | **1.8 h^{b} (50 °C)** | **8.72** | Zhang et al. 2013 |
| sp. DPEase | **7.5-8.0** | 60 | 28:72 | **1.6 h (60 °C)** | **16** | Zhu et al. 2012 |
| *A. tumefaciens* DPEase | **8.0** | 50 | 32:68 (30 °C) | **8.90 min (55 °C)** | **85** | Kim et al. 2006 |
| | | | 33:67 (40 °C) | **3.99 min (60 °C)** | | |
| *A. tumefaciens* DPEase mutant of S213C | **NR^{a}** | NR | NR | **0.46 h (55 °C)** | **101** | Choi et al. 2011 |
| *A. tumefaciens* DPEase mutant of I33L | NR | NR | NR | **1.06 h (55 °C)** | **105** | Choi et al. 2011 |
| *A. tumefaciens* DPEase mutant of S213C+I33L | **NR** | NR | NR | **4.4 h (55 °C)** | **134** | Choi et al. 2011 |
| *Rhizobium* DTEase | **9.0-9.5** | 50 | 23:77 | **NR** | **NR** | Maruta et al. 2010 |
| *P. cichorii* DTEase | **7.5** | 60 | 20:80 (30 °C) | **1 h (50 °C)** | **NR** | Itoh et al. 1994 |
| *R*. *sphaeroides* DTEase | **9.0** | 40 | 23:77 (40 °C) | **NR** | **NR** | Zhang et al. 2009 |

| | | | | | | |
|---|---|---|---|---|---|---|
| *^{a}* NR, not reported. *^{b}* The half-life values were converted from the original references with the unit of min. *^{a}* The value was converted from the orginal reference with the unit of mM⁻¹ s⁻¹. | | | | | | |

**SEQUENCE LISTING TABLE**

| **SEQ ID No** | **Description** |
|---|---|
| 1 | Nucleic acid sequence of the parent D-psicose 3-epimerase from *Clostridium cellulolyticum* |
| 2 | Amino acid sequence of the parent D-psicose 3-epimerase from *Clostridium cellulolyticum* |
| 3 | Nucleic acid sequence of the D-psicose 3-epimerase variant derived from *Clostridium cellulolyticum* |
| 4 | Amino acid sequence of the D-psicose 3-epimerase variant derived from *Clostridium cellulolyticum* |
| 5 | Amino acid sequence of *Clostridium sp.* DPEase |
| 6 | Amino acid sequence of *C. scindens* DPEase |
| 7 | Amino acid sequence of *A. tumefaciens* DPEase |
| 8 | Amino acid sequence of *Ruminococcus* sp. DPEase |
| 9 | Amino acid sequence of *C. bolteae* DPEase |
| 10 | Amino acid sequence of *P. cichorii* DTEase |
| 11 | Amino acid sequence of *R*. *sphaeroides* DTEase |
| 12-20 | Primers |

## Claims

1. A variant of a parent D-psicose 3-epimerase, wherein the variant comprises a substitution of a Glycine residue by a Serine residue at a position corresponding to the position 211 in SEQ ID No 2 compared to the parent D-psicose 3-epimerase; and wherein the variant has a D-psicose 3-epimerase activity.

2. The variant according to claim 1, wherein the variant has one or several following features:
a. a lower pH optimum compared to the parent D-psicose 3-epimerase, preferably in the range of 6 to 7; and/or
b. a higher catalysis efficiency to the substrate D-fructose compared to the parent-psicose 3-epimerase, preferably at least twice higher; and/or
c. a longer half-life at 60°C compared to the parent-psicose 3-epimerase.

3. The variant according to claim 1 or 2, wherein the variant has an amino acid sequence having 35 % of identity or higher with SEQ ID No 2, preferably 60 % of identity or higher, more preferably at least 70, 75, 80, 85, 90, 95 % of identity or higher.

4. The variant according to any one of claims 1-3, wherein the parent D-psicose 3-epimerase is selected from a D-tagatose 3-epimerase from *Pseudomonas cichorii,* a D-psicose 3-epimerase from *Agrobacterium tumefaciens,* a D-psicose 3-epimerase from *Clostridium sp,* a D-psicose 3-epimerase from *Clostridium scindens,* a D-psicose 3-epimerase from *Clostridium bolteae,* a D-psicose 3-epimerase from *Ruminococcus sp,* and a D-psicose 3-epimerase from *Clostridium cellulolyticum.*

5. The variant according to any one of claims 1-4, wherein the parent D-psicose 3-epimerase is the D-psicose 3-epimerase from *Clostridium cellulolyticum.*

6. The variant according to claim 5, wherein the variant comprises or consists of the amino acid sequence of SEQ ID No 4 or an amino acid sequence having 90 or 95 % of identity with SEQ ID No 4 or higher with a residue Ser at position 211.

7. An isolated nucleic acid encoding a variant according to any one of claims 1-6.

8. A recombinant expression vector comprising a nucleic acid according to claim 7.

9. A recombinant host cell comprising a nucleic acid according to claim 7 or a recombinant expression vector according to claim 8.

10. The recombinant host cell according to claim 9, wherein the host cell is a GRAS strain (Generally Recognized As Safe), preferably *Bacillus subtilis.*

11. A method for producing a D-psicose 3-epimerase variant comprising culturing the recombinant host cell according to claim 9 or 10, and optionally recovering the produced D-psicose 3-epimerase variant from the resulting culture.

12. A method for producing D-psicose comprising contacting a variant according to any one of claims 1 to 6 with D-fructose in conditions suitable for the D-psicose 3-epimerase activity and optionally recovering the produced D-psicose.

13. The method of claim 12, wherein the D-fructose is previously or simultaneously produced by a glucose isomerase from D-glucose.

14. An enzymatic composition comprising a D-psicose 3-epimerase variant according to any one of claims 1 to 6 and an additional enzyme, in particular a glucose isomerase.

15. Use of a D-psicose 3-epimerase variant according to any one of claims 1 to 6 or a host cell according to claim 9 or 10 for producing D-psicose.

16. Use of a host cell according to claim 10 for preparing a food product.

17. A food product comprising a host cell according to claim 10.


SEQUENCE LISTING

<110>  ROQUETTE FRERES

<120>  Improved variant of D-psicose 3-epimerase and uses
thereof

<130>  B1628EP

<160>  20

<170>  PatentIn version 3.3

<210>  1
<211>  879
<212>  DNA
<213>  Clostridium cellulolyticum


<220>
<221>  CDS
<222>  (1)..(879)

<400>  1
atg aaa cat ggt ata tac tac gca tat tgg gaa caa gaa tgg gaa
gct      48
Met Lys His Gly Ile Tyr Tyr Ala Tyr Trp Glu Gln Glu Trp Glu
Ala
1               5                   10                  15


gat tac aaa tac tat att gag aag gtt gca aag ctt ggt ttt gat
att      96
Asp Tyr Lys Tyr Tyr Ile Glu Lys Val Ala Lys Leu Gly Phe Asp
Ile
            20                  25                  30


cta gag att gca gct tca ccg cta cct ttt tac agt gac att cag
att     144
Leu Glu Ile Ala Ala Ser Pro Leu Pro Phe Tyr Ser Asp Ile Gln
Ile
        35                  40                  45


aat gag ctc aag gca tgt gcc cat ggc aat gga att aca ctt acg
gta     192
Asn Glu Leu Lys Ala Cys Ala His Gly Asn Gly Ile Thr Leu Thr
Val
    50                  55                  60


ggc cat ggg cct agt gca gaa caa aac ctg tct tct ccc gac ccc
gat     240
Gly His Gly Pro Ser Ala Glu Gln Asn Leu Ser Ser Pro Asp Pro
Asp
65                  70                  75                  80


att cgc aaa aat gct aaa gct ttt tat acc gat tta ctc aaa cga
ctt     288
Ile Arg Lys Asn Ala Lys Ala Phe Tyr Thr Asp Leu Leu Lys Arg
Leu

85 90 95

tac aag ctg gat gta cat ttg ata ggt ggg gct tta tat tct tat
tgg 336
Tyr Lys Leu Asp Val His Leu Ile Gly Gly Ala Leu Tyr Ser Tyr
Trp
100 105 110

ccg ata gat tac aca aag aca att gat aaa aaa ggc gat tgg gaa
cgc 384
Pro Ile Asp Tyr Thr Lys Thr Ile Asp Lys Lys Gly Asp Trp Glu
Arg
115 120 125

agc gtt gaa agt gtt cga gaa gtt gct aag gtg gcc gaa gcc tgt
gga 432
Ser Val Glu Ser Val Arg Glu Val Ala Lys Val Ala Glu Ala Cys
Gly
130 135 140

gtg gat ttc tgc cta gag gtt ctt aat aga ttt gag aat tat tta
att 480
Val Asp Phe Cys Leu Glu Val Leu Asn Arg Phe Glu Asn Tyr Leu
Ile
145 150 155
160

aac aca gca caa gag ggt gta gat ttt gta aaa cag gtt gac cat
aac 528
Asn Thr Ala Gln Glu Gly Val Asp Phe Val Lys Gln Val Asp His
Asn
165 170 175

aat gta aag gta atg ctt gat acc ttc cac atg aat att gag gaa
gat 576
Asn Val Lys Val Met Leu Asp Thr Phe His Met Asn Ile Glu Glu
Asp
180 185 190

agt atc gga ggt gca atc agg act gcg ggc tct tac ttg gga cat
tta 624
Ser Ile Gly Gly Ala Ile Arg Thr Ala Gly Ser Tyr Leu Gly His
Leu
195 200 205

cac act ggc gaa tgt aat cgt aaa gtt ccc ggc aga gga aga att
cca 672
His Thr Gly Glu Cys Asn Arg Lys Val Pro Gly Arg Gly Arg Ile
Pro
210 215 220

tgg gta gaa att ggt gag gct ctt gct gac ata ggt tat aac ggt
agt 720
Trp Val Glu Ile Gly Glu Ala Leu Ala Asp Ile Gly Tyr Asn Gly
Ser

225                 230                 235
240

gtt gtt atg gaa cct ttt gtt aga atg ggc gga act gtc gga tct
aat      768

Val Val Met Glu Pro Phe Val Arg Met Gly Gly Thr Val Gly Ser
Asn

                245                 250                 255

att aag gtt tgg cgt gac att agt aac ggt gca gat gag aaa atg
ctg      816

Ile Lys Val Trp Arg Asp Ile Ser Asn Gly Ala Asp Glu Lys Met
Leu

            260                 265                 270

gat aga gaa gca cag gcc gca ctt gat ttc tcc aga tat gta tta
gaa      864

Asp Arg Glu Ala Gln Ala Ala Leu Asp Phe Ser Arg Tyr Val Leu
Glu

        275                 280                 285

tgt cat aaa cac tcc
879

Cys His Lys His Ser

    290

<210>  2
<211>  293
<212>  PRT
<213>  Clostridium cellulolyticum

<400>  2

Met Lys His Gly Ile Tyr Tyr Ala Tyr Trp Glu Gln Glu Trp Glu
Ala

1               5                   10                  15

Asp Tyr Lys Tyr Tyr Ile Glu Lys Val Ala Lys Leu Gly Phe Asp
Ile

            20                  25                  30

Leu Glu Ile Ala Ala Ser Pro Leu Pro Phe Tyr Ser Asp Ile Gln
Ile

        35                  40                  45

Asn Glu Leu Lys Ala Cys Ala His Gly Asn Gly Ile Thr Leu Thr
Val

    50                  55                  60

Asp Gly His Gly Pro Ser Ala Glu Gln Asn Leu Ser Ser Pro Asp Pro
65                  70                  75                  80

Leu Ile Arg Lys Asn Ala Lys Ala Phe Tyr Thr Asp Leu Leu Lys Arg
                85                  90                  95

Trp Tyr Lys Leu Asp Val His Leu Ile Gly Gly Ala Leu Tyr Ser Tyr
            100                 105                 110

Arg Pro Ile Asp Tyr Thr Lys Thr Ile Asp Lys Lys Gly Asp Trp Glu
        115                 120                 125

Gly Ser Val Glu Ser Val Arg Glu Val Ala Lys Val Ala Glu Ala Cys
    130                 135                 140

Ile Val Asp Phe Cys Leu Glu Val Leu Asn Arg Phe Glu Asn Tyr Leu
145 150 155
160

Asn Asn Thr Ala Gln Glu Gly Val Asp Phe Val Lys Gln Val Asp His
                165                 170                 175

Asp Asn Val Lys Val Met Leu Asp Thr Phe His Met Asn Ile Glu Glu
            180                 185                 190

Leu Ser Ile Gly Gly Ala Ile Arg Thr Ala Gly Ser Tyr Leu Gly His
        195                 200                 205

Pro His Thr Gly Glu Cys Asn Arg Lys Val Pro Gly Arg Gly Arg Ile
    210                 215                 220

Ser Trp Val Glu Ile Gly Glu Ala Leu Ala Asp Ile Gly Tyr Asn Gly
225 230 235

240

Val Val Met Glu Pro Phe Val Arg Met Gly Gly Thr Val Gly Ser
Asn
245 250 255

Ile Lys Val Trp Arg Asp Ile Ser Asn Gly Ala Asp Glu Lys Met
Leu
260 265 270

Asp Arg Glu Ala Gln Ala Ala Leu Asp Phe Ser Arg Tyr Val Leu
Glu
275 280 285

Cys His Lys His Ser
    290

<210> 3
<211> 879
<212> DNA
<213> artificial sequence

<220>
<223> CC-DPEase variant

<220>
<221> CDS
<222> (1)..(879)

<400> 3
atg aaa cat ggt ata tac tac gca tat tgg gaa caa gaa tgg gaa
gct 48
Met Lys His Gly Ile Tyr Tyr Ala Tyr Trp Glu Gln Glu Trp Glu
Ala
1 5 10 15

gat tac aaa tac tat att gag aag gtt gca aag ctt ggt ttt gat
att 96
Asp Tyr Lys Tyr Tyr Ile Glu Lys Val Ala Lys Leu Gly Phe Asp
Ile
20 25 30

cta gag att gca gct tca ccg cta cct ttt tac agt gac att cag
att 144
Leu Glu Ile Ala Ala Ser Pro Leu Pro Phe Tyr Ser Asp Ile Gln
Ile
35 40 45

aat gag ctc aag gca tgt gcc cat ggc aat gga att aca ctt acg
gta 192

Asn Glu Leu Lys Ala Cys Ala His Gly Asn Gly Ile Thr Leu Thr
Val
50 55 60

ggc cat ggg cct agt gca gaa caa aac ctg tct tct ccc gac ccc
gat 240
Gly His Gly Pro Ser Ala Glu Gln Asn Leu Ser Ser Pro Asp Pro
Asp
65 70 75 80

att cgc aaa aat gct aaa gct ttt tat acc gat tta ctc aaa cga
ctt 288
Ile Arg Lys Asn Ala Lys Ala Phe Tyr Thr Asp Leu Leu Lys Arg
Leu
85 90 95

tac aag ctg gat gta cat ttg ata ggt ggg gct tta tat tct tat
tgg 336
Tyr Lys Leu Asp Val His Leu Ile Gly Gly Ala Leu Tyr Ser Tyr
Trp
100 105 110

ccg ata gat tac aca aag aca att gat aaa aaa ggc gat tgg gaa
cgc 384
Pro Ile Asp Tyr Thr Lys Thr Ile Asp Lys Lys Gly Asp Trp Glu
Arg
115 120 125

agc gtt gaa agt gtt cga gaa gtt gct aag gtg gcc gaa gcc tgt
gga 432
Ser Val Glu Ser Val Arg Glu Val Ala Lys Val Ala Glu Ala Cys
Gly
130 135 140

gtg gat ttc tgc cta gag gtt ctt aat aga ttt gag aat tat tta
att 480
Val Asp Phe Cys Leu Glu Val Leu Asn Arg Phe Glu Asn Tyr Leu
Ile
145 150 155
160

aac aca gca caa gag ggt gta gat ttt gta aaa cag gtt gac cat
aac 528
Asn Thr Ala Gln Glu Gly Val Asp Phe Val Lys Gln Val Asp His
Asn
165 170 175

aat gta aag gta atg ctt gat acc ttc cac atg aat att gag gaa
gat 576
Asn Val Lys Val Met Leu Asp Thr Phe His Met Asn Ile Glu Glu
Asp
180 185 190

agt atc gga ggt gca atc agg act gcg ggc tct tac ttg gga cat
tta 624

Ser Ile Gly Gly Ala Ile Arg Thr Ala Gly Ser Tyr Leu Gly His
Leu
195 200 205

cac act agc gaa tgt aat cgt aaa gtt ccc ggc aga gga aga att
cca 672
His Thr Ser Glu Cys Asn Arg Lys Val Pro Gly Arg Gly Arg Ile
Pro
210 215 220

tgg gta gaa att ggt gag gct ctt gct gac ata ggt tat aac ggt
agt 720
Trp Val Glu Ile Gly Glu Ala Leu Ala Asp Ile Gly Tyr Asn Gly
Ser
225 230 235
240

gtt gtt atg gaa cct ttt gtt aga atg ggc gga act gtc gga tct
aat 768
Val Val Met Glu Pro Phe Val Arg Met Gly Gly Thr Val Gly Ser
Asn
245 250 255

att aag gtt tgg cgt gac att agt aac ggt gca gat gag aaa atg
ctg 816
Ile Lys Val Trp Arg Asp Ile Ser Asn Gly Ala Asp Glu Lys Met
Leu
260 265 270

gat aga gaa gca cag gcc gca ctt gat ttc tcc aga tat gta tta
gaa 864
Asp Arg Glu Ala Gln Ala Ala Leu Asp Phe Ser Arg Tyr Val Leu
Glu
275 280 285

tgt cat aaa cac tcc
879
Cys His Lys His Ser
    290

<210> 4
<211> 293
<212> PRT
<213> artificial sequence

<220>
<223> Synthetic Construct

<400> 4

Met Lys His Gly Ile Tyr Tyr Ala Tyr Trp Glu Gln Glu Trp Glu
Ala
1 5 10 15

Ile Asp Tyr Lys Tyr Tyr Ile Glu Lys Val Ala Lys Leu Gly Phe Asp
            20                  25                  30

Ile Leu Glu Ile Ala Ala Ser Pro Leu Pro Phe Tyr Ser Asp Ile Gln
        35                  40                  45

Val Asn Glu Leu Lys Ala Cys Ala His Gly Asn Gly Ile Thr Leu Thr
    50                  55                  60

Asp Gly His Gly Pro Ser Ala Glu Gln Asn Leu Ser Ser Pro Asp Pro
65                  70                  75                  80

Leu Ile Arg Lys Asn Ala Lys Ala Phe Tyr Thr Asp Leu Leu Lys Arg
                85                  90                  95

Trp Tyr Lys Leu Asp Val His Leu Ile Gly Gly Ala Leu Tyr Ser Tyr
            100                 105                 110

Arg Pro Ile Asp Tyr Thr Lys Thr Ile Asp Lys Lys Gly Asp Trp Glu
        115                 120                 125

Gly Ser Val Glu Ser Val Arg Glu Val Ala Lys Val Ala Glu Ala Cys
    130                 135                 140

Ile Val Asp Phe Cys Leu Glu Val Leu Asn Arg Phe Glu Asn Tyr Leu
145 150 155
160

Asn Asn Thr Ala Gln Glu Gly Val Asp Phe Val Lys Gln Val Asp His
                165                 170                 175

Asn Val Lys Val Met Leu Asp Thr Phe His Met Asn Ile Glu Glu

Asp
180 185 190

Ser Ile Gly Gly Ala Ile Arg Thr Ala Gly Ser Tyr Leu Gly His
Leu
195 200 205

His Thr Ser Glu Cys Asn Arg Lys Val Pro Gly Arg Gly Arg Ile
Pro
210 215 220

Trp Val Glu Ile Gly Glu Ala Leu Ala Asp Ile Gly Tyr Asn Gly
Ser
225 230 235
240

Val Val Met Glu Pro Phe Val Arg Met Gly Gly Thr Val Gly Ser
Asn
245 250 255

Ile Lys Val Trp Arg Asp Ile Ser Asn Gly Ala Asp Glu Lys Met
Leu
260 265 270

Asp Arg Glu Ala Gln Ala Ala Leu Asp Phe Ser Arg Tyr Val Leu
Glu
275 280 285

Cys His Lys His Ser
    290

<210> 5
<211> 292
<212> PRT
<213> Clostridium sp.

<400> 5

Met Lys His Gly Ile Tyr Tyr Ala Tyr Trp Glu Gln Glu Trp Glu
Ala
1 5 10 15

Asp Tyr Lys Tyr Tyr Ile Glu Lys Val Ala Lys Leu Gly Phe Asp
Ile
20 25 30

Ile Leu Glu Ile Ala Ala Ser Pro Leu Pro Phe Tyr Ser Asp Asn Gln
        35                  40                  45

Val Asn Glu Leu Lys Ala Cys Ala Arg Gly Asn Gly Ile Thr Leu Thr
    50                  55                  60

Tyr Gly His Gly Pro Ser Ala Glu Gln Asn Leu Ser Ser Pro Asp Pro
65                  70                  75                  80

Leu Ile Arg Lys Asn Ala Lys Ala Phe Tyr Thr Asp Leu Leu Lys Arg
                85                  90                  95

Trp Tyr Lys Leu Asp Val His Leu Ile Gly Gly Ala Ile Tyr Ser Tyr
            100                 105                 110

Arg Pro Val Asp Tyr Thr Lys Thr Ile Asp Lys Lys Gly Asp Trp Glu
        115                 120                 125

Gly Ser Val Glu Ser Val Arg Glu Val Ala Gln Val Ala Glu Ala Cys
    130                 135                 140

Ile Val Asp Phe Cys Leu Glu Val Leu Asn Arg Phe Glu Asn Tyr Leu
145 150 155
160

Asp Asn Thr Ala Gln Glu Gly Val Asp Phe Val Lys Gln Val Gly His
                165                 170                 175

Asp Asn Val Lys Val Met Leu Asp Thr Phe His Met Asn Ile Glu Glu
            180                 185                 190

Leu Ser Ile Gly Gly Ala Ile Arg Thr Ala Gly Ser Tyr Leu Gly His

        195                 200                 205

His Thr Gly Glu Cys Asn Arg Lys Val Pro Gly Lys Gly Arg Ile
Pro
210 215 220

Trp Ile Glu Ile Gly Glu Ala Leu Ala Asp Ile Gly Tyr Asn Gly
Ser
225 230 235
240

Val Val Met Glu Pro Phe Val Arg Met Gly Gly Thr Val Gly Ser
Asn
245 250 255

Ile Lys Val Trp Arg Asp Ile Ser Asn Gly Ala Asp Glu Glu Lys
Leu
260 265 270

Asp Arg Glu Ala Gln Ala Ala Leu Asn Phe Ser Arg Tyr Val Leu
Gly
275 280 285

Asn Arg Lys Leu
    290

<210> 6
<211> 289
<212> PRT
<213> Clostridium scindens

<400> 6

Met Lys His Gly Ile Tyr Tyr Ala Tyr Trp Glu Gln Glu Trp Ala
Ala
1 5 10 15

Asp Tyr Lys Arg Tyr Val Glu Lys Ala Ala Lys Leu Gly Phe Asp
Ile
20 25 30

Leu Glu Val Gly Ala Ala Pro Leu Pro Asp Tyr Ser Ala Gln Glu
Val
35 40 45

Lys Glu Leu Lys Lys Cys Ala Asp Asp Asn Gly Ile Gln Leu Thr
Ala
50 55 60

Gly Tyr Gly Pro Ala Phe Asn His Asn Met Gly Ser Ser Asp Pro
Lys
65 70 75 80

Ile Arg Glu Glu Ala Leu Gln Trp Tyr Lys Arg Leu Phe Glu Val
Met
85 90 95

Ala Gly Leu Asp Ile His Leu Ile Gly Gly Ala Leu Tyr Ser Tyr
Trp
100 105 110

Pro Val Asp Phe Ala Thr Ala Asn Lys Glu Glu Asp Trp Lys His
Ser
115 120 125

Val Glu Gly Met Gln Ile Leu Ala Pro Ile Ala Ser Gln Tyr Gly
Ile
130 135 140

Asn Leu Gly Met Glu Val Leu Asn Arg Phe Glu Ser His Ile Leu
Asn
145 150 155
160

Thr Ser Glu Glu Gly Val Lys Phe Val Thr Glu Val Gly Met Asp
Asn
165 170 175

Val Lys Val Met Leu Asp Thr Phe His Met Asn Ile Glu Glu Ser
Ser
180 185 190

Ile Gly Asp Ala Ile Arg His Ala Gly Lys Leu Leu Gly His Phe
His
195 200 205

Thr Gly Glu Cys Asn Arg Met Val Pro Gly Lys Gly Arg Thr Pro
Trp
210 215 220

Arg Glu Ile Gly Asp Ala Leu Arg Glu Ile Glu Tyr Asp Gly Thr
Val
225 230 235
240

Val Met Glu Pro Phe Val Arg Met Gly Gly Gln Val Gly Ser Asp
Ile
245 250 255

Lys Val Trp Arg Asp Ile Ser Lys Gly Ala Gly Glu Asp Arg Leu
Asp
260 265 270

Glu Asp Ala Arg Arg Ala Val Glu Phe Gln Arg Tyr Met Leu Glu
Trp
275 280 285

Lys

<210> 7
<211> 288
<212> PRT
<213> Agrobacterium tumefaciens

<400> 7

Met Lys His Gly Ile Tyr Tyr Ser Tyr Trp Glu His Glu Trp Ser
Ala
1 5 10 15

Lys Phe Gly Pro Tyr Ile Glu Lys Val Ala Lys Leu Gly Phe Asp
Ile
20 25 30

Ile Glu Val Ala Ala His His Ile Asn Glu Tyr Ser Asp Ala Glu
Leu
35 40 45

Ala Thr Ile Arg Lys Ser Ala Lys Asp Asn Gly Ile Ile Leu Thr
Ala
50 55 60

Gly Ile Gly Pro Ser Lys Thr Lys Asn Leu Ser Ser Glu Asp Ala

Ala
65                  70                  75                  80

Val Arg Ala Ala Gly Lys Ala Phe Phe Glu Arg Thr Leu Ser Asn
Val
                85                  90                  95

Ala Lys Leu Asp Ile His Thr Ile Gly Gly Ala Leu His Ser Tyr
Trp
            100                 105                 110

Pro Ile Asp Tyr Ser Gln Pro Val Asp Lys Ala Gly Asp Tyr Ala
Arg
        115                 120                 125

Gly Val Glu Gly Ile Asn Gly Ile Ala Asp Phe Ala Asn Asp Leu
Gly
    130                 135                 140

Ile Asn Leu Cys Ile Glu Val Leu Asn Arg Phe Glu Asn His Val
Leu
145                 150                 155
160

Asn Thr Ala Ala Glu Gly Val Ala Phe Val Lys Asp Val Gly Lys
Asn
                165                 170                 175

Asn Val Lys Val Met Leu Asp Thr Phe His Met Asn Ile Glu Glu
Asp
            180                 185                 190

Ser Phe Gly Asp Ala Ile Arg Thr Ala Gly Pro Leu Leu Gly His
Phe
        195                 200                 205

His Thr Gly Glu Ser Asn Arg Arg Val Pro Gly Lys Gly Arg Met
Pro
    210                 215                 220

Trp His Glu Ile Gly Leu Ala Leu Arg Asp Ile Asn Tyr Thr Gly
Ala
225                 230                 235

240

Asp Val Ile Met Glu Pro Phe Val Lys Thr Gly Gly Thr Ile Gly Ser
                245                 250                 255

Met Ile Lys Val Trp Arg Asp Leu Ser Gly Gly Ala Asp Ile Ala Lys
            260                 265                 270

Gly Asp Glu Asp Ala Arg Asn Ala Leu Ala Phe Ser Arg Phe Val Leu
        275                 280                 285

<210> 8
<211> 291
<212> PRT
<213> Ruminococcus sp.

<400> 8

Gly Met Lys Tyr Gly Ile Tyr Tyr Ala Tyr Trp Glu Lys Glu Trp Asn
1               5                   10                  15

Ile Asp Tyr Lys Tyr Tyr Ile Asp Lys Ile Ser Lys Leu Gly Phe Asp
            20                  25                  30

Gln Leu Glu Ile Ser Cys Gly Ala Phe Ser Asp Tyr Tyr Thr Lys Asp
        35                  40                  45

Leu Glu Leu Ile Asp Ile Gly Lys Tyr Ala Lys Glu Lys Gly Val Thr
    50                  55                  60

Glu Thr Ala Gly Tyr Gly Pro His Phe Asn Glu Ser Leu Ser Ser Ser
65                  70                  75                  80

Arg Pro Asn Thr Gln Lys Gln Ala Ile Ser Phe Trp Lys Glu Thr Leu
                85                  90                  95

Gly Lys Leu Lys Leu Met Asp Ile His Ile Val Gly Gly Ala Leu Tyr
            100                 105                 110

Leu Tyr Trp Pro Val Asp Tyr Ser Lys Pro Phe Asp Lys Lys Arg Asp
        115                 120                 125

Glu Glu Asn Ser Ile Lys Asn Met Lys Ile Ile Ser Gln Tyr Ala Glu
    130                 135                 140

Tyr Tyr Asp Ile Met Met Gly Met Glu Val Leu Asn Arg Phe Glu Gly
145 150 155
160

Gly Met Leu Asn Thr Cys Asp Glu Ala Leu Ala Tyr Val Glu Glu Val
                165                 170                 175

Glu Ser Ser Asn Val Gly Val Met Leu Asp Thr Phe His Met Asn Ile
            180                 185                 190

Tyr Glu Asp Asn Ile Ala Ala Ala Ile Arg Lys Ala Gly Asp Arg Leu
        195                 200                 205

Met His Phe His Ile Gly Glu Gly Asn Arg Lys Val Pro Gly Lys Gly
    210                 215                 220

Gln Leu Pro Trp Asn Glu Ile Gly Gln Ala Leu Arg Asp Ile Asn Tyr
225 230 235
240

Gly His Ala Ala Val Met Glu Pro Phe Val Met Gln Gly Gly Thr Val
                245                 250                 255

Val His Asp Ile Lys Ile Trp Arg Asp Ile Ile Gly Asn Cys Ser Glu

            260                 265                 270

Val
Thr Leu Asp Met Asp Ala Gln Ser Ala Leu His Phe Val Lys His
    275                 280                 285

Phe Glu Val
    290

<210> 9
<211> 301
<212> PRT
<213> Clostridium bolteae

<400> 9

Tyr
Met Arg Tyr Phe Lys Glu Glu Val Ala Gly Met Lys Tyr Gly Ile
1 5 10 15

Met
Phe Ala Tyr Trp Thr Lys Glu Trp Phe Ala Asp Tyr Lys Lys Tyr
        20                  25                  30

Ala
Asp Lys Val Ser Ala Leu Gly Phe Asp Val Leu Glu Ile Ser Cys
    35                  40                  45

Arg
Ala Leu Arg Asp Val Tyr Thr Thr Lys Glu Gln Leu Ile Glu Leu
50                  55                  60

Pro
Glu Tyr Ala Lys Glu Lys Gly Leu Val Leu Thr Ala Gly Tyr Gly
65 70 75 80

Arg
Thr Lys Ala Glu Asn Leu Cys Ser Glu Asp Pro Glu Ala Val Arg
            85                  90                  95

Asp
Ala Met Thr Phe Phe Lys Asp Leu Leu Pro Lys Leu Gln Leu Met
        100                 105                 110

Phe Ile His Ile Leu Gly Gly Gly Leu Tyr Ser Tyr Trp Pro Val Asp
        115                 120                 125

Asn Thr Ile Asn Asn Asp Lys Gln Gly Asp Arg Ala Arg Ala Val Arg
    130                 135                 140

Gly Leu Arg Glu Leu Ser Lys Thr Ala Glu Glu Cys Asp Val Val Leu
145 150 155
160

Glu Met Glu Val Leu Asn Arg Tyr Glu Gly Tyr Ile Leu Asn Thr Cys
                165                 170                 175

Ile Glu Ala Ile Asp Phe Val Asp Glu Ile Gly Ser Ser His Val Lys
            180                 185                 190

Asp Met Leu Asp Thr Phe His Met Asn Ile Glu Glu Thr Asn Met Ala
        195                 200                 205

Glu Ala Ile Arg Lys Ala Gly Asp Arg Leu Gly His Leu His Leu Gly
    210                 215                 220

Ile Gln Asn Arg Leu Val Pro Gly Lys Gly Ser Leu Pro Trp Ala Glu
225 230 235
240

Glu Gly Gln Ala Leu Arg Asp Ile Asn Tyr Gln Gly Ala Ala Val Met
                245                 250                 255

Trp Pro Phe Val Met Gln Gly Gly Thr Ile Gly Ser Glu Ile Lys Val
            260                 265                 270

Ala Arg Asp Met Val Pro Asp Leu Ser Glu Glu Ala Leu Asp Arg Asp
        275                 280                 285

Lys Gly Ala Leu Glu Phe Cys Arg His Val Phe Gly Ile
    290                 295                 300

<210>  10
<211>  290
<212>  PRT
<213>  Pseudomonas cichorii

<400>  10

Met Asn Lys Val Gly Met Phe Tyr Thr Tyr Trp Ser Thr Glu Trp
Met
1               5                   10                  15

Val Asp Phe Pro Ala Thr Ala Lys Arg Ile Ala Gly Leu Gly Phe
Asp
            20                  25                  30

Leu Met Glu Ile Ser Leu Gly Glu Phe His Asn Leu Ser Asp Ala
Lys
        35                  40                  45

Lys Arg Glu Leu Lys Ala Val Ala Asp Asp Leu Gly Leu Thr Val
Met
    50                  55                  60

Cys Cys Ile Gly Leu Lys Ser Glu Tyr Asp Phe Ala Ser Pro Asp
Lys
65                  70                  75                  80

Ser Val Arg Asp Ala Gly Thr Glu Tyr Val Lys Arg Leu Leu Asp
Asp
                85                  90                  95

Cys His Leu Leu Gly Ala Pro Val Phe Ala Gly Leu Thr Phe Cys
Ala
            100                 105                 110

Trp Pro Gln Ser Pro Pro Leu Asp Met Lys Asp Lys Arg Pro Tyr
Val
        115                 120                 125

Asp Asp Arg Ala Ile Glu Ser Val Arg Arg Val Ile Lys Val Ala Glu
    130                 135                 140

Trp Tyr Gly Ile Ile Tyr Ala Leu Glu Val Val Asn Arg Phe Glu Gln
145 150 155
160

Asp Leu Cys Asn Asp Ala Lys Glu Ala Ile Ala Phe Ala Asp Ala Val
                165                 170                 175

Glu Ser Pro Ala Cys Lys Val Gln Leu Asp Thr Phe His Met Asn Ile
            180                 185                 190

Gly Glu Thr Ser Phe Arg Asp Ala Ile Leu Ala Cys Lys Gly Lys Met
        195                 200                 205

Arg His Phe His Leu Gly Glu Ala Asn Arg Leu Pro Pro Gly Glu Gly
    210                 215                 220

Asp Leu Pro Trp Asp Glu Ile Phe Gly Ala Leu Lys Glu Ile Gly Tyr
225 230 235
240

Ser Gly Thr Ile Val Met Glu Pro Phe Met Arg Lys Gly Gly Ser Val
                245                 250                 255

Glu Arg Ala Val Gly Val Trp Arg Asp Met Ser Asn Gly Ala Thr Asp
            260                 265                 270

Lys Glu Met Asp Glu Arg Ala Arg Arg Ser Leu Gln Phe Val Arg Asp
        275                 280                 285

Leu Ala
    290

<210> 11
<211> 295
<212> PRT
<213> Rhodobacter sphaeroides

<400> 11

Met Lys Asn Pro Val Gly Ile Ile Ser Met Gln Phe Ile Arg Pro
Phe
1 5 10 15

Thr Ser Glu Ser Leu His Phe Leu Lys Lys Ser Arg Ala Leu Gly
Phe
20 25 30

Asp Phe Ile Glu Leu Leu Val Pro Glu Pro Glu Asp Gly Leu Asp
Ala
35 40 45

Ala Glu Val Arg Arg Ile Cys Glu Gly Glu Gly Leu Gly Leu Val
Leu
50 55 60

Ala Ala Arg Val Asn Leu Gln Arg Ser Ile Ala Ser Glu Glu Ala
Ala
65 70 75 80

Ala Arg Ala Gly Gly Arg Asp Tyr Leu Lys Tyr Cys Ile Glu Ala
Ala
85 90 95

Glu Ala Leu Gly Ala Thr Ile Val Gly Gly Pro Leu Tyr Gly Glu
Pro
100 105 110

Leu Val Phe Ala Gly Arg Pro Pro Phe Pro Trp Thr Ala Glu Gln
Ile
115 120 125

Ala Thr Arg Ala Ala Arg Thr Val Glu Gly Leu Ala Glu Val Ala
Pro
130 135 140

Leu Ala Ala Ser Ala Gly Lys Val Phe Gly Leu Glu Pro Leu Asn

Arg
145 150 155
160

Phe Glu Thr Asp Ile Val Asn Thr Thr Ala Gln Ala Ile Glu Val
Val
165 170 175

Asp Ala Val Gly Ser Pro Gly Leu Gly Val Met Leu Asp Thr Phe
His
180 185 190

Met Asn Met Glu Glu Arg Ser Ile Pro Asp Ala Ile Arg Ala Thr
Gly
195 200 205

Ala Arg Leu Val His Phe Gln Ala Asn Glu Asn His Arg Gly Phe
Pro
210 215 220

Gly Thr Gly Thr Met Asp Trp Thr Ala Ile Ala Arg Ala Leu Gly
Gln
225 230 235
240

Ala Gly Tyr Ala Gly Pro Val Ser Leu Glu Pro Phe Arg Arg Asp
Asp
245 250 255

Glu Arg Val Ala Leu Pro Ile Ala His Trp Arg Ala Pro His Glu
Asp
260 265 270

Glu Asp Glu Lys Leu Arg Ala Gly Leu Gly Leu Ile Arg Ser Ala
Ile
275 280 285

Thr Leu Ala Glu Val Thr His
    290                 295

<210> 12
<211> 27
<212> DNA
<213> artificial sequence

<220>
<223>  primer

<400>  12
catttacaca ctagcgaatg taatcgt
27

<210>  13
<211>  27
<212>  DNA
<213>  artificial sequence

<220>
<223>  primer

<400>  13
catttacaca ctgccgaatg taatcgt
27

<210>  14
<211>  27
<212>  DNA
<213>  artificial sequence

<220>
<223>  primer

<400>  14
catttacaca ctgacgaatg taatcgt
27

<210>  15
<211>  27
<212>  DNA
<213>  artificial sequence

<220>
<223>  primer

<400>  15
catttacaca ctcgcgaatg taatcgt
27

<210>  16
<211>  27
<212>  DNA
<213>  artificial sequence

<220>
<223>  primer

<400>  16
catttacaca cttgggaatg taatcgt
27

<210>  17
<211>  27
<212>  DNA

<213>  artificial sequence

<220>
<223>  primer

<400>  17
catttacaca ctctcgaatg taatcgt
27


<210>  18
<211>  30
<212>  DNA
<213>  artificial sequence

<220>
<223>  primer

<400>  18
agtgtgtaaa tgtcccaagt aagagcccgc
30


<210>  19
<211>  29
<212>  DNA
<213>  artificial sequence

<220>
<223>  primer

<400>  19
cgccatatga aacatggtat atactacgc
29


<210>  20
<211>  25
<212>  DNA
<213>  artificial sequence

<220>
<223>  primer

<400>  20
cgcggatcct tgttagccgg atctc
25